# EUROPEAN PATENT APPLICATION

(11) **EP 4 591 801 A1**
(43) Date of publication of application: **30.07.2025**
(21) Application number: 23881038.6
(22) Date of filing: 01.03.2023
(51) Int. Cl.: A61B 8/00, A61M 5/00

(54) **FULL-AUTOMATIC FOAMING APPARATUS AND METHOD**

(30) Priority: 24.10.2022 CN 202211300528
(71) Applicant: Carefree Heartbeat Medical Technology (Shenzhen) Co., Ltd., Shenzhen, Guangdong 518118 (CN)
(72) Inventor: KANG, Libiao, Shenzhen, Guangdong 518118 (CN); LIAO, Haiyan, Shenzhen, Guangdong 518118 (CN); ZHENG, Zhenkun, Shenzhen, Guangdong 518118 (CN)
(74) Representative: Hertin und Partner Rechts- und Patentanwälte PartG mbB
(86) International application number: PCT/CN2023/079110
(87) International publication number: WO 2024/087435

(57) **Abstract**

The present invention provides a full-automatic foaming apparatus and method. The full-automatic foaming apparatus comprises: an instruction input apparatus, used for receiving a user instruction and outputting an instruction signal; an automatic bolus apparatus, used for performing automatic bolus to form a suspension, and injecting the suspension; a gas blowing apparatus, used for receiving gas exhaled by a user, a first measurement apparatus, used for measuring the pressure inside a liquid injection apparatus of the automatic bolus apparatus and outputting a first pressure signal; a second measurement apparatus, used for measuring the pressure inside the gas blowing apparatus and outputting a second pressure signal; and a control apparatus, used for enabling the automatic bolus apparatus to perform bolus on the basis of the instruction signal, further used for enabling the automatic bolus apparatus to stop bolus on the basis of the first pressure signal, and further used for enabling the automatic bolus apparatus to inject the suspension on the basis of the second pressure signal. According to the present invention, by means of mutual cooperation of the apparatuses, full automation of the foaming and injection processes of a foaming test is realized, the level of automation is high, and the diagnosis accuracy is improved.

## Description

### TECHNICAL FIELD

The present invention relates to the technical field of medical devices, and specifically relates to a full-automatic foaming apparatus and a method.

### BACKGROUND ART

Currently, the primary diagnostic techniques for patent foramen ovale (PFO) include the transthoracic echocardiography (TTE), transesophageal echocardiography (TEE), and transcranial Doppler (TCD) foaming tests. All three technical methods rely on the foaming test to aid in diagnosis.

The current operation process of the foaming test is as follows: 1) preparing two 10 ml syringes with saline and air respectively, wherein three ports of the triple valve are connected to two syringes and infusion needles respectively; 2) drawing a small amount of blood by the infusion needle and allowing the blood to enter one of the syringes by the triple valve; 3) connecting the two syringes by the triple valve, and manually pushing the two syringes reciprocally, so as to form a well-mixed suspension (generally pushing not less than 10 times); 4) injecting into the human body as a control group; and 5) repeating steps 1-4 above; injecting the prepared suspension when the patient performs the Valsalva maneuver (let the patient take a deep breath, hold it, and then exhale forcefully); and observing whether there is any microembolus signal on the TCD test or ultrasonic instrument.

As a result, the operation process of the current foaming test has the following defects. It needs the medical personnel to operate the triple valve; the medical personnel needs to push back and forth several times for foaming; and the medical personnel needs to observe the TCD test or the ultrasonic instrument when injecting the suspension, i.e., the entire operation process requires the manual participation, and at least two medical personnel need to participate, which is time-consuming and laborious, and has a high work intensity. The entire diagnostic process needs the medical personnel to observe the TCD test or the ultrasonic instrument throughout the process and the close cooperation of the patient, but the medical personnel or the patient does not cooperate properly, which results in the incorrect diagnostic result, so that the diagnosis accuracy is low. At present, the method of repeated diagnosis is commonly used to improve the accuracy of the diagnosis result, which is time-consuming and laborious.

### SUMMARY

In order to overcome the defects of the above prior art, the present invention provides a full-automatic foaming apparatus and a method.

The specific technical solutions are as follows.

A full-automatic foaming apparatus includes:
an instruction input device, configured to receive a user instruction and output an instruction signal;
an automatic pushing and injecting device, configured to automatically push to form a suspension and inject the suspension;
a blowing device, configured to receive gas exhaled by the user;
a first detection device, connected to the automatic pushing and injecting device, and configured to detect a pressure within a liquid injection device of the automatic pushing and injecting device and to output a first pressure signal;
a second detection device, connected to the blowing device, and configured to detect a pressure within the blowing device and to output a second pressure signal; and
a control device, electrically connected to the instruction input device, the automatic pushing and injecting device, the first detection device, and the second detection device; configured to receive the instruction signal and allow the automatic pushing and injecting device to push based on the instruction signal; further configured to receive the first pressure signal and allow the automatic pushing and injecting device to stop pushing based on the first pressure signal; and further configured to receive the second pressure signal and allow the automatic pushing and injecting device to inject the suspension based on the second pressure signal.

In a specific embodiment, the instruction input device includes a start-stop button, wherein the start-stop button is electrically connected to the control device;
configured to receive a start instruction and to output a start instruction signal to the control device, so that the control device drives the automatic pushing and injecting device for pushing and injecting; and
further configured to receive a stop instruction and to output a stop instruction signal to the control device, so that the control device drives the automatic pushing and injecting device for stopping pushing and injecting.

In a specific embodiment, a power source is further included, wherein the power source is electrically connected to the control device via a switching circuit, and the instruction input device includes a power button and an emergency stop button, wherein
the power button is electrically connected to the switching circuit, configured to receive a user power-on instruction and to output a power-on signal to conduct the switching circuit, so that the control device starts to operate, and further configured to receive a user power-off instruction and to output a power-off signal to disconnect the switching circuit, so that the control device stops operating; and
the emergency stop button is electrically connected to the switching circuit, and configured to receive a user emergency stop instruction and to output an emergency stop signal to disconnect the switching circuit, so that the control device stops operating.

In a specific embodiment, a display device is further included, and the display device is provided with an instruction signal display interface, a first pressure signal display interface, and a second pressure signal display interface, wherein
the display device is electrically connected to the control device, configured to receive an instruction signal output by the control device and to display it to the instruction signal display interface, to receive a first pressure signal output by the control device and to display it to the first pressure signal display interface, and to receive a second pressure signal output by the control device and to display it to the second pressure signal display interface.

In a specific embodiment, the automatic pushing and injecting device includes a base, and the base is provided thereon with the following components:
a triple valve;
a first liquid injection device, communicated to a first outlet of the triple valve;
a second liquid injection device, communicated to a second outlet of the triple valve;
an intravenous needle, communicated to a third outlet of the triple valve;
a first automatic push device, connected to the first liquid injection device, and configured to drive a piston rod of the first liquid injection device to move reciprocally;
a second automatic push device, connected to the second liquid injection device, and configured to drive a piston rod of the second liquid injection device to move reciprocally; and
an automatic rotating device, configured to drive a valve knob of the triple valve to rotate, so as to communicate two outlets of the triple valve.

In a specific embodiment, the control device is electrically connected to the first automatic push device, the second automatic push device, and the automatic rotating device;
configured to drive the automatic rotating device to rotate, and to drive the first automatic push device and the second automatic push device to push based on the instruction signal;
further configured to drive the first automatic push device and the second automatic push device to stop pushing, and to drive the automatic rotating device to rotate based on the first pressure signal; and
further configured to drive the second automatic push device to inject the suspension based on the second pressure signal.

In a specific embodiment, the automatic rotating device includes a power device and a rotation rod connected to each other, and the rotation rod is configured to contact the valve knob and to rotate the valve knob driven by the power device, wherein
the first automatic push device includes a first driving device and a first driving rod, wherein the first driving device is connected to the piston rod of the first liquid injection device by the first driving rod, and
the second automatic push device includes a second driving device and a second driving rod, wherein the second driving device is connected to the piston rod of the second liquid injection device by the second driving rod.

A full-automatic foaming method is applied to the full-automatic foaming apparatus, including:
receiving the user instruction and outputting the instruction signal by the instruction input device;
receiving the instruction signal to control the automatic pushing and injecting device to automatically push to form the suspension by the control device;
detecting the pressure within the liquid injection device of the automatic pushing and injecting device by the first detection device and outputting the first pressure signal;
receiving the first pressure signal to control the automatic pushing and injecting device to stop pushing by the control device;
detecting the pressure within the blowing device by the second detection device and outputting the second pressure signal; and
receiving the second pressure signal to control the automatic pushing and injecting device to inject the suspension by the control device.

In a specific embodiment, the automatic pushing and injecting device includes the base, and the triple valve, the first liquid injection device, the second liquid injection device, the intravenous needle, the first automatic push device, the second automatic push device, and the automatic rotating device arranged on the base, wherein the first automatic push device is connected to the first liquid injection device, and the first liquid injection device is communicated with the first outlet of the triple valve; the second automatic push device is connected to the second liquid injection device, and the second liquid injection device is communicated with the second outlet of the triple valve; and the intravenous needle is communicated with the third outlet of the triple valve, wherein
the step of receiving the instruction signal to control the automatic pushing and injecting device to automatically push to form the suspension by the control device includes:
receiving the instruction signal by the control device to control the automatic rotating device to rotate the valve knob of the triple valve, so as to communicate the first liquid injection device and the second liquid injection device; controlling the first automatic push device to drive the piston rod of the first liquid injection device to move reciprocally; and controlling the second automatic push device to drive the piston rod of the second liquid injection device to move reciprocally.

In a specific embodiment, the step of receiving the first pressure signal to control the automatic pushing and injecting device to stop pushing by the control device includes:
receiving the first pressure signal to control the second automatic push device to stop pushing by the control device;
controlling the first automatic push device to drive the piston rod of the first liquid injection device by the control device, so as to store the suspension into the second liquid injection device; and
controlling the first automatic push device to stop pushing by the control device after the suspension is completely stored in the second liquid injection device.

The present invention has at least the following beneficial effects.

The present invention provides a full-automatic foaming apparatus and a method, wherein the full-automatic foaming apparatus includes: an instruction input device, configured to receive a user instruction and output an instruction signal; an automatic pushing and injecting device, configured to automatically push to form a suspension and inject the suspension; a blowing device, configured to receive gas exhaled by the user; a first detection device, connected to the automatic pushing and injecting device, and configured to detect a pressure within a liquid injection device of the automatic pushing and injecting device and to output a first pressure signal; a second detection device, connected to in the blowing device, and configured to detect a pressure within the blowing device and to output a second pressure signal; and a control device, electrically connected to the instruction input device, the automatic pushing and injecting device, the first detection device, and the second detection device; configured to receive the instruction signal and allow the automatic pushing and injecting device to push based on the instruction signal; further configured to receive the first pressure signal and allow the automatic pushing and injecting device to stop pushing based on the first pressure signal; and further configured to receive the second pressure signal and allow the automatic pushing and injecting device to inject the suspension based on the second pressure signal. In the present invention, the instruction input device, the automatic pushing and injecting device, the blowing device, the first detection device, the second detection device, and the control device cooperate with each other, which realizes the full automation of the foaming process and the injection process of the foaming test and provides a high-level automation, thereby freeing the hands of medical personnel and reducing the work intensity of the medical personnel; and at the same time, it overcomes the defects that the diagnostic accuracy is low due to the difference in the pushing duration and the difference in the individual operation method in the manual operation, so as to improve the diagnostic accuracy rate.

Further, a full-automatic foaming method is further provided, applied to the full-automatic foaming apparatus, including: receiving the user instruction and outputting the instruction signal by the instruction input device; receiving the instruction signal to control the automatic pushing and injecting device to automatically push to form the suspension by the control device; detecting the pressure within the liquid injection device of the automatic pushing and injecting device by the first detection device and outputting the first pressure signal; receiving the first pressure signal to control the automatic pushing and injecting device to stop pushing by the control device; detecting the pressure within the blowing device by the second detection device and outputting the second pressure signal; and receiving the second pressure signal to control the automatic pushing and injecting device to inject the suspension by the control device. The high level of automation reduces the human operation in the foaming test phase, and reduces the work intensity of the medical personnel, which enhances their experience; and at the same time, it overcomes the defects that the diagnostic accuracy is low due to the difference in the pushing duration and the difference in the individual operation method in the manual operation, so as to improve the diagnostic accuracy rate.

### BRIEF DESCRIPTION OF DRAWINGS

In order to more clearly illustrate the technical solutions in the embodiments of the present invention, the drawings described in the embodiments will be briefly introduced below. It is obvious that the drawings in the following description are only some embodiments of the present invention, and for persons of ordinary skill in the art, other drawings can be obtained based on these drawings without inventive efforts.
FIG. 1 is a structure schematic diagram of a full-automatic foaming apparatus provided in Embodiment 1;
FIG. 2 is another structure schematic diagram of a full-automatic foaming apparatus provided in Embodiment 1;
FIG. 3 is another structure schematic diagram of a full-automatic foaming apparatus provided in Embodiment 1;
FIG. 4 is another structure schematic diagram of a full-automatic foaming apparatus provided in Embodiment 1;
FIG. 5 is another structure schematic diagram of a full-automatic foaming apparatus provided in Embodiment 1;
FIG. 6 is a partially enlarged diagram of region A in FIG. 5;
FIG. 7 is a partially enlarged diagram of region B in FIG. 6;
FIG. 8 is another structure schematic diagram of a full-automatic foaming apparatus provided in Embodiment 1;
FIG. 9 is another structure schematic diagram of a full-automatic foaming apparatus provided in Embodiment 1;
FIG. 10 shows a flow diagram of a full-automatic foaming method provided in Embodiment 2;
FIG. 11 shows another flow diagram of a full-automatic foaming method provided in Embodiment 2; and
FIG. 12 shows another flow diagram of a full-automatic foaming method provided in Embodiment 2.

### Reference numbers:

1-base; 2-first liquid injection device; 3-second liquid injection device; 4-triple valve; 5-first automatic push device; 6-automatic rotating device; 8-instruction input device; 9-control device; 10-display device; 11-mounting base; 12-mounting part; 13-first fixing member; 14-second fixing member; 15-covering member; 16-gas tube; 17-handle; 18-handle hook; 19-blowing member; 20-case; 21-bracket; 41-first outlet; 42-second outlet; 43-third outlet; 44-valve knob; 51-first driving device; 52-first driving rod; 53-driving plate; 54-ball-head rod end joint bearing; 55-ball joint; 56-single elbow joint; 57-buffer block; 61-power device; 62-rotation rod; 63-accommodation groove; 71-first detection device; 72-second detection device; 81-power button; 82-emergency stop button; 83-start-stop button; 91-switching circuit; 111-first placing groove; 112-second placing groove; 113-third placing groove; 114-groove; 151-hinge; 152-tubular body limiting groove; 153-injection port limiting groove; 154-observation window; x-first direction; and y-second direction.

### DETAILED DESCRIPTION OF EMBODIMENTS

Various embodiments of the present invention will be described more completely below. The present invention may have various embodiments, and the adaptations and changes may be made therein. However, it should be understood that it is not to limit the various embodiments of the present invention to the particular embodiments disclosed herein, but the present invention should be understood to include all adjustments, equivalents, and/or options that fall within the spirit and scope of the various embodiments of the present invention.

In the following, the terms "include" or "may include", which may be used in various embodiments of the present invention to indicate the existence of the disclosed function, operation, or component, and do not limit the addition of one or more functions, operations, or components. Additionally, the terms "include", "have", and their cognates used in various embodiments of the present invention, are intended only to represent a particular feature, number, step, operation, element, component, or combination of the foregoing, and should not be understood as precluding the existence of one or more other features, numbers, steps, operations, elements, components, or combinations of the foregoing, or improving the possibility of one or more other features, numbers, steps, operations, elements, components, or combinations of the foregoing.

In various embodiments of the present invention, the expression "or" or "at least one of A or/and B" includes any or all combinations of the words listed at the same time. For example, the expression "A or B" or "at least one of A or/and B" may include A, may include B, or may include both A and B.

The expressions used in various embodiments of the present invention (such as "first" and "second") may modify various components in various embodiments, although the corresponding components may not be limited. For example, the above expression does not limit the order and/or importance of the components. The above expressions are used only for the purpose of distinguishing one component from the others. For example, the first user device and the second user device indicate different user devices, although both are user devices. For example, without departing from the scope of various embodiments of the present invention, the first component may be referred to as the second component, and similarly, the second component may be referred to as the first component.

It should be noted that in the present invention, unless other expressly specifications and limitations, the terms "mount", "connect", and "fix" are to be understood in a broad sense, e.g. it may be a fixed connection, a detachable connection, or an integral connection; it can be a mechanical connection or an electrical connection; and it can be a direct connection, an indirect connection through an intermediate medium, or a communication inside two elements. For a person of ordinary skill in the art, the specific meaning of the above terms in the present invention can be understood according to specific situations.

In the present invention, it is to be understood by those of ordinary skill in the art that the terms in the text indicating the orientation or positional relationship are based on the orientation or positional relationships based on the drawings, which are only to facilitate the description of the present invention and simplify the description, and are not to indicate or imply that the device or element referred to must have a particular orientation, or be constructed and operated with a particular orientation, and therefore cannot to be understood as limitations of the present invention.

### Embodiment 1

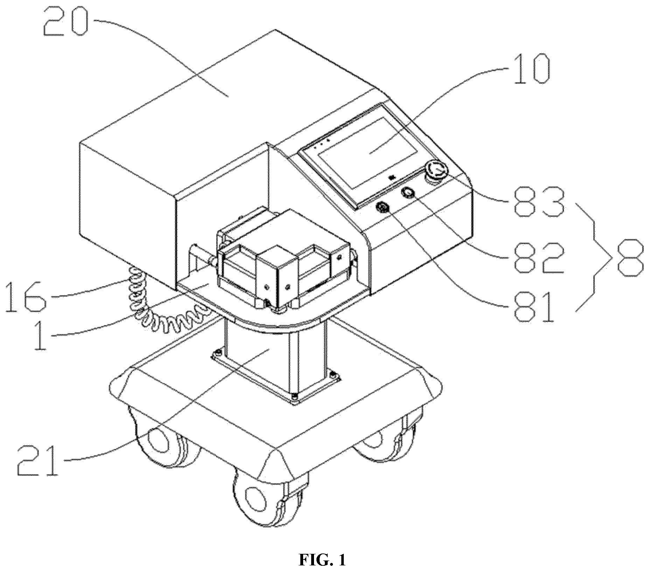

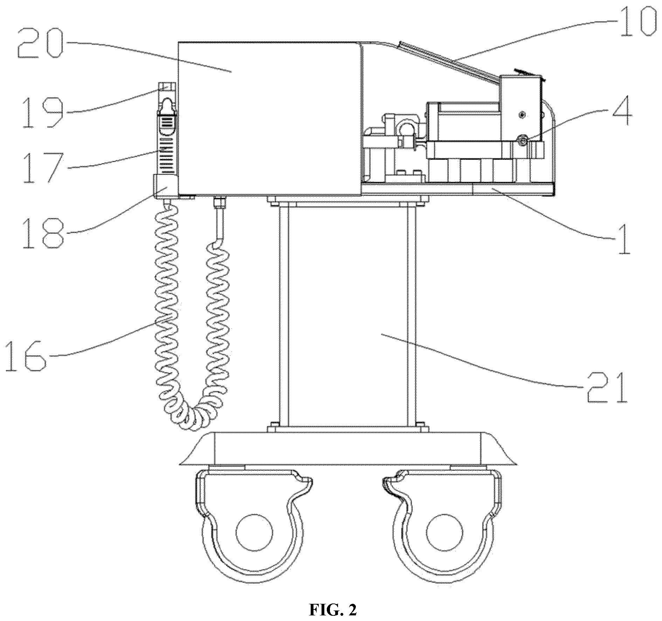

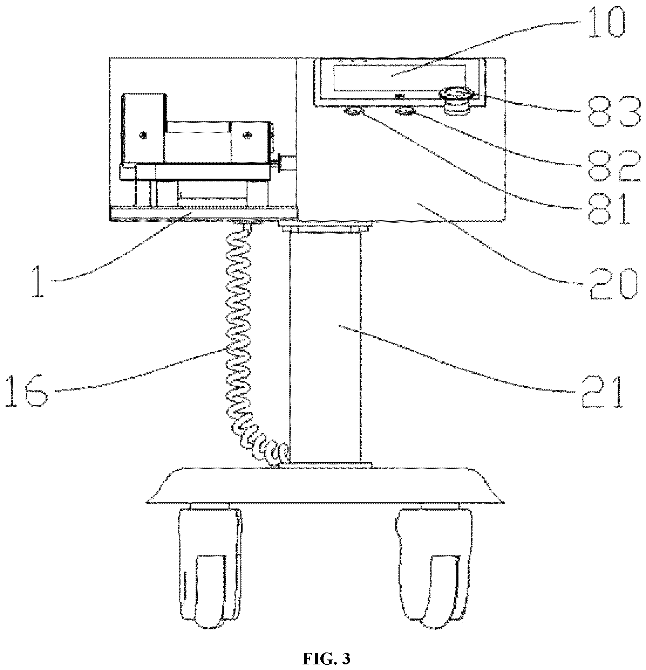

As shown in FIG. 1 to FIG. 9, the present invention provides a full-automatic foaming apparatus, including:
an instruction input device 8, configured to receive a user instruction and output an instruction signal;
an automatic pushing and injecting device, configured to automatically push to form a suspension and to inject the suspension;
a blowing device, configured to receive gas exhaled by the user;
a first detection device 71, connected to the automatic pushing and injecting device, and configured to detect a pressure within a liquid injection device of the automatic pushing and injecting device and to output a first pressure signal;
a second detection device 72, connected to the blowing device, and configured to detect a pressure within the blowing device and to output a second pressure signal; and
a control device 9, electrically connected to the instruction input device 8, the automatic pushing and injecting device, the first detection device 71, and the second detection device 72; configured to receive the instruction signal and allow the automatic pushing and injecting device to push based on the instruction signal; further configured to receive the first pressure signal and allow the automatic pushing and injecting device to stop pushing based on the first pressure signal; and further configured to receive the second pressure signal and allow the automatic pushing and injecting device to inject the suspension based on the second pressure signal.

The current foaming test needs manual reciprocal pushing of 2 syringes, so as to make a well-mixed suspension (generally pushing not less than 10 times) of the saline, air, and blood, and it needs to prepare two suspensions (one for the control group and one for the test group), so that the medical personnel needs to spend a lot of time and energy on the pushing stage, and the work intensity is high.

Moreover, the triple valve further needs to be controlled by the medical personnel during the pushing stage to communicate with the corresponding channel, so that the water, air, and blood are mixed, which requires the participation of the medical personnel during the whole process.

Furthermore, the foaming test needs the participation of at least two medical personnel (one to push and the other medical personnel to observe), which results in high labor costs.

Additionally, it is not easy to control the pushing duration through the manual operation. Due to the difference in the personal operating proficiency and operating habit of medical personnel, errors are likely to occur during the pushing process, which results in a lower diagnostic accuracy.

Moreover, some patients are unable to complete the standard Valsalva maneuver (let the patient take a deep breath, hold it, and then exhale forcefully) due to their age, which results in an incorrect diagnostic result, so that the diagnosis accuracy is not high. At present, the method of repeated diagnosis is commonly used to improve the accuracy of the diagnosis result, which is time-consuming and laborious.

In the present invention, the instruction input device 8, the automatic pushing and injecting device, the blowing device, the first detection device 71, the second detection device 72, and the control device 9 cooperate with each other, which realizes the full automation of the foaming process and the injection process of the foaming test and provides a high-level automation, thereby freeing hands of the medical personnel, reducing the work intensity of the medical personnel, and improving the experience of the medical personnel; and at the same time, it overcomes the defects that the diagnostic accuracy is not high due to the difference in the pushing duration, the difference in the individual operation method, and the inadequate patient cooperation in the manual operation, so as to improve the diagnostic accuracy rate.

After the control device 9 controls the automatic pushing and injecting device to start pushing and foaming according to the user instructions received by the instruction input device 8, the first detection device 71 detects in real time the pressure within the liquid injection device of the automatic pushing and injecting device and outputs the first pressure signal to the control device 9. When the pressure within the liquid injection device reaches a preset value, the control device 9 controls the automatic pushing and injecting device to stop working, and the foaming process ends. It realizes fully automatic foaming by machine without manual participation, and the automation level is high.

After the foaming process ends, the second detection device 72 detects the pressure of the exhaled gas of the user and outputs the second pressure signal to the control device 9. When the pressure of the exhaled gas of the user reaches a preset value (e.g., 40 mmHg), the control device 9 controls the automatic pushing and injecting device to inject the suspension. It realizes the full automation of the injection process of the foaming test without the manual participation, and the automation level is high. It uses the blowing-sensing technology instead of requiring the user to perform the Valsalva maneuver (let the patient take a deep breath, hold it, and then exhale forcefully) to accurately assess right atrial pressure, and it is to automatically push the foaming agent when the pressure reaches the preset value, which avoids the poor test result since some patients are unable to complete the standard Valsalva maneuver due to their age, so as to provide a wide range of applications and a high accuracy of the test result.

Specifically, the control device is preferably a single chip microcontroller. Optionally, the PLC (programmable logic controller) and the photoelectric switch can also be used, but not limited to this.

As shown in FIG. 1, FIG. 3 and FIG. 4, the full-automatic foaming apparatus further includes a power source, wherein the power source is electrically connected to the control device 9 via the switching circuit 91, and the instruction input device 8 includes a power button 81 and an emergency stop button 82.

The power button 81 is electrically connected to the switching circuit 91, configured to receive a user power-on instruction and to output a power-on signal to conduct the switching circuit 91, so that the control device 9 starts to operate, and further configured to receive a user power-off instruction and to output a power-off signal to disconnect the switching circuit 91, so that the control device 9 stops operating.

The emergency stop button 82 is electrically connected to the switching circuit 91, and configured to receive a user emergency stop instruction and to output an emergency stop signal to disconnect the switching circuit 91, so that the control device 9 stops operating.

The present invention provides the power button 81, so that the user can turn on or off the full-automatic foaming apparatus by pressing the power button 81, which is easy to operate; and by providing the emergency stop button 82, the user can cut off the power source of the full-automatic foaming apparatus by pressing the emergency stop button 82 in case of emergency, which is highly safe.

Specifically, the full-automatic foaming apparatus further includes a backup power source, and the backup power source is also electrically connected to the control device 9 via the switching circuit 91, so that the foaming operation and the emergency stop operation can be carried out even in the case of a sudden power failure.

As shown in FIG. 1, FIG. 3 and FIG. 4, the instruction input device 8 further includes a start-stop button 83, wherein the start-stop button 83 is electrically connected to the control device 9. The start-stop button 83 is configured to receive a start instruction and to output a start instruction signal to the control device 9, so that the control device 9 drives the automatic pushing and injecting device to push and inject based on the start instruction signal, and is further configured to receive a stop instruction and to output a stop instruction signal to the control device 9, so that the control device 9 drives the automatic pushing and injecting device to stop pushing and injecting based on the stop instruction signal. The user only needs to press the start-stop button 83, the full-automatic foaming apparatus is able to start the pushing and foaming operation, so that the level of automation is high and the user experience is better.

Specifically, a voice device is further included, wherein the voice device is electrically connected to the control device 9. The control device 9 receives the signal output from the instruction input device 8 and then generates a voice broadcast signal, and the voice device receives the voice broadcast signal and then performs a voice broadcast, which facilitates the user to monitor the foaming test process.

As shown in FIG. 1, FIG. 2, FIG. 3, and FIG. 4, a display device 10 is further included, wherein the display device 10 is provided with an instruction signal display interface, a first pressure signal display interface, and a second pressure signal display interface.

The display device 10 is electrically connected to the control device 9, configured to receive an instruction signal output by the control device 9 and to display it to the instruction signal display interface, to receive a first pressure signal output by the control device 9 and to display it to the first pressure signal display interface, and to receive a second pressure signal output by the control device 9 and to display it to the second pressure signal display interface. The user can monitor the whole process of the operating program by the display device 10, which enhances human-computer interaction and improves the user experience.

As shown in FIG. 1, FIG. 2, and FIG. 3, the automatic pushing and injecting device includes a base 1; a triple valve 4 arranged on the base 1; a first liquid injection device 2, communicated to a first outlet 41 of the triple valve; a second liquid injection device 3, communicated to a second outlet 42 of the triple valve; an intravenous needle (not shown in the figure), communicated to a third outlet 43 of the triple valve; a first automatic push device 5, connected to the first liquid injection device 2, and configured to drive a piston rod of the first liquid injection device 2 to move reciprocally; a second automatic push device, connected to the second liquid injection device 3, and configured to drive a piston rod of the second liquid injection device 3 to move reciprocally; and an automatic rotating device 6, configured to drive a valve knob 44 of the triple valve to rotate, so as to communicate two outlets of the triple valve 4.

The base 1 is provided with a case 20, and the case 20 covers the base 1. The intravenous needle, the first liquid injection device 2, the second liquid injection device 3, the triple valve 4, the first automatic push device 5, the second automatic push device, the automatic rotating device 6, and the control device 9 are arranged on the base 1. The instruction input device 8 and the display device 10 are arranged on the case 20. A liftable bracket 21 is arranged at the bottom of the base 1, so as to adjust the height of the base 1 to adapt to hospital beds of different heights.

Specifically, in the embodiment, the bracket 21 utilizes a lifting column controlled by a stepper motor. Optionally, the bracket 21 with the lifting functions including pneumatic spring lifting, hydraulic lifting, and mechanical lifting can also be used.

Specifically, the bracket 21 is provided with silent brake pulleys at the bottom, which are easy to be pushed and fixed by the medical personnel.

Specifically, the blowing device includes a blowing member 19 and a gas tube 16 connected to each other, wherein the gas tube 16 passes through the base 1 to connect to the second detection device 72, and the blowing member 19 and the gas tube 16 are connected by a handle 17. The case 20 is further provided with a handle hook 18, which facilitates fixing the handle 17. Preferably, the handle 17 is provided with a filtering device for filtering moisture and particles in the exhaled gas of the user, which effectively protects the second detection device 72 and improves the service life of the second detection device 72.

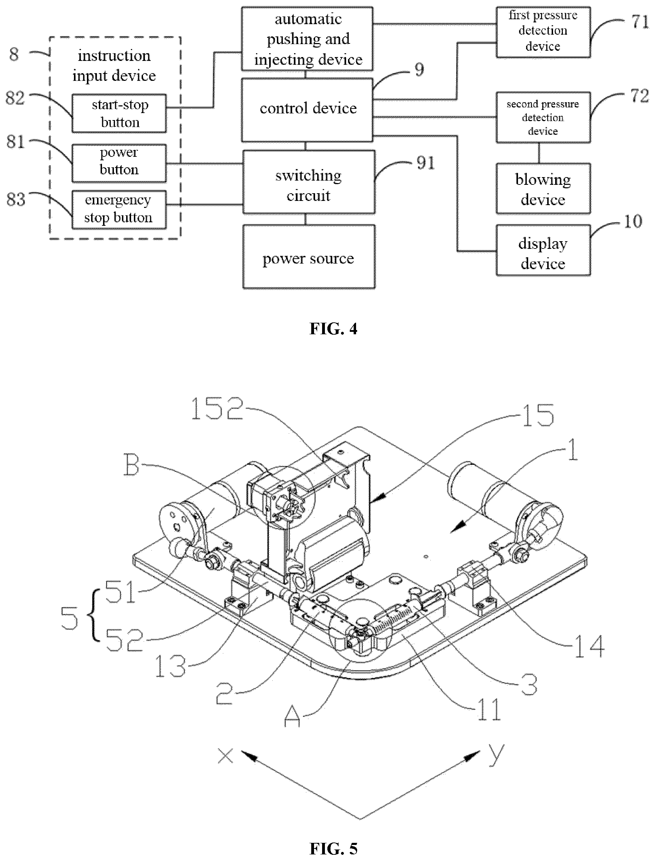

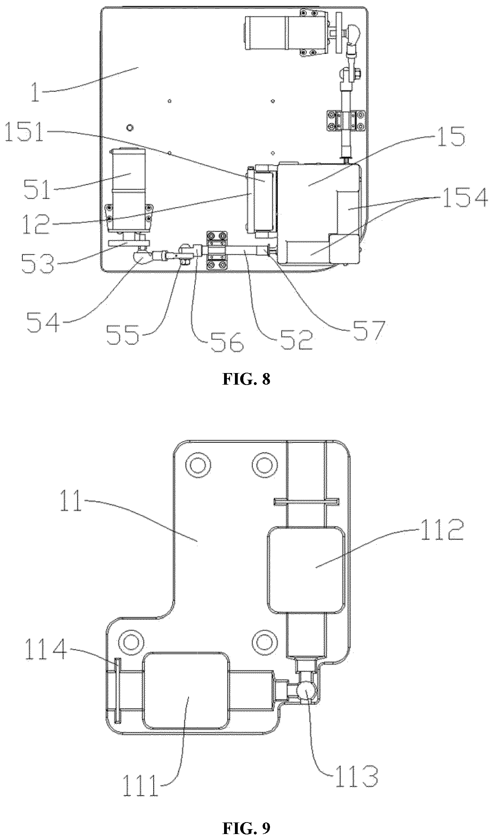

As shown in FIG. 5 and FIG. 8, in the embodiment, the base 1 is in a square-like structure with a rounded corner. The base 1 is provided with a mounting base 11 near the rounded corner, and the mounting base 11 is configured to mount the triple valve 4, the first liquid injection device 2, and the second liquid injection device 3. The mounting base 11 is provided with a first placing groove 111 and a second placing groove 112. The tube body of the first liquid injection device 2 is located in the first placing groove 111, and a tube body of the second liquid injection device 3 is located in the second placing groove 112. The mounting base 11 is further provided with a third placing groove 113, wherein the third placing groove 113 is communicated with the first placing groove 111 and the second placing groove 112, respectively, and the triple valve 4 is located in the third placing groove 113.

Specifically, the first liquid injection device 2 and the second liquid injection device 3 adopt liquid injection devices with a capacity of 20 ml, so as to reduce the pushing stroke, and improve the reliability of pushing the liquid injection device by using the automatic push device. Optionally, it can also be a liquid injection device with a capacity between 10 ml and 50 ml.

Specifically, as shown in FIG. 8, the end of the first placing groove 111 is provided with a groove 114 for accommodating a tube wing of the first liquid injection device 2, which avoids the synchronized movement of the tube body of the first liquid injection device 2 when the first automatic push device 5 drives the piston rod of the first liquid injection device 2 to move reciprocally, thereby affecting the foaming effect of the suspension. The structure of the second placing groove 112 is the same as the structure of the first placing groove 111, which will not be repeated herein.

As shown in FIG. 5 and FIG. 8, the first placing groove 111 is arranged in a first direction x; the second placing groove 112 is arranged in a second direction y; and an angle between the first direction x and the second direction y is preset. In the embodiment, the first direction x is perpendicular to the second direction y; the first placing groove 111 is perpendicular to the second placing groove 112; and the first liquid injection device 2 is perpendicular to the second liquid injection device 2.

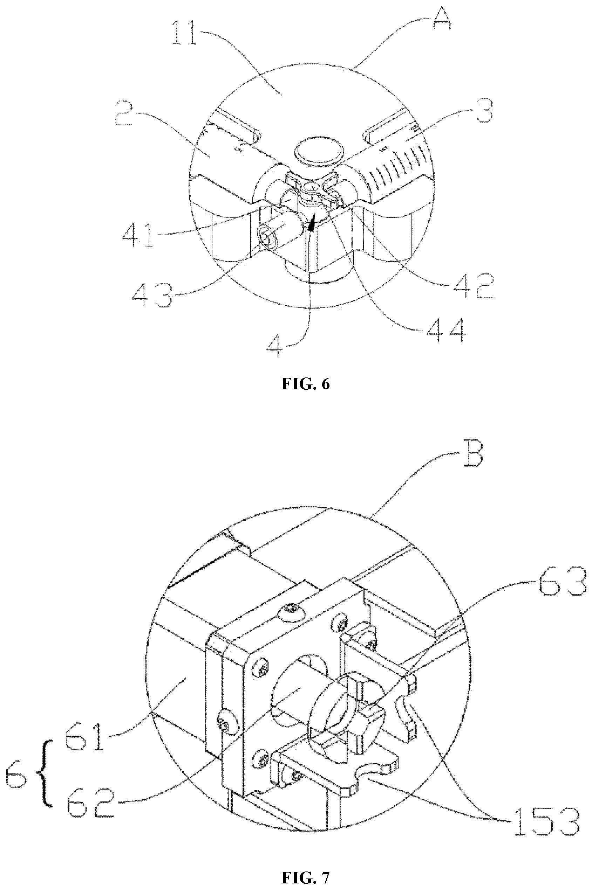

As shown in FIG. 6, the triple valve 4 has a first outlet 41, a second outlet 42, and a third outlet 43, wherein the first outlet 41 is communicated with an injection port of the first liquid injection device 2; the second outlet 42 is communicated with an injection port of the second liquid injection device 3; and the third outlet 43 is communicated with the intravenous needle.

Specifically, the intravenous needle is preferably an indwelling needle.

Specifically, the intravenous needle can be connected to the third outlet 43 by an extension tube.

Specifically, the first liquid injection device 2 and the first outlet 41, and the second liquid injection device 2 and the second outlet 42, are connected by Luer tapers. Preferably, the Luer taper with a threaded structure is adopted.

As shown in FIG. 5 and FIG. 8, a covering member 15 is further included. The covering member 15 is rotatably arranged on the base 1 for covering the mounting base 11 and the first liquid injection device 2, the second liquid injection device 3, and the triple valve 4 arranged on the mounting base 11, and also for fixing the first liquid injection device 2 and the second liquid injection device 3 to avoid movement thereof when covering. It is also used to rotate to expose the mounting base 11, the first liquid injection device 2, the second liquid injection device 3, and the triple valve 4 arranged on the mounting base 11 under the action of the external force.

During the pushing stage of the foaming test, the covering member 15 covers the base 1, and the covering member 15 and the base 1 are located on two sides of the first liquid injection device 2 and the second liquid injection device 3, which facilitates the movement of the piston rod of the liquid injection device driven by the automatic push device 5, and avoids the movement of the first liquid injection device 2 and the second liquid injection device 3. During the reagent preparation stage of the foaming test, the covering member 15 rotates to expose the mounting base 11, which facilitates the installation of the first liquid injection device 2, the second liquid injection device 3, and the triple valve 4. After the pushing stage is completed, the covering member 15 rotates to expose the mounting base 11, which facilitates the disassembly of the first liquid injection device 2, the second liquid injection device 3, and the triple valve 4. As shown in FIG. 8, a mounting part 12 for mounting the covering member 15 is arranged on the base 1, and a rotational connection structure is arranged between the mounting part 12 and the covering member 15. In the embodiment, the rotational connection structure includes an electric motor and a hinge 71, wherein the electric motor drives the hinge 71 to drive the covering member 15 to rotate relative to the base 1. In the embodiment, the covering member 15 is rotated by the motorized method, which drives the manual operation by the machine, so as to further improve the automation level and free hands, thereby reducing the work intensity of medical personnel.

Optionally, the mounting part 12 and the covering member 15 can also be connected by a rotary shaft, and the medical personnel rotates the covering member 15 by manual method.

As shown in FIG. 5, FIG. 7, and FIG. 9, a first limiting groove corresponding to the first liquid injection device 2 and a second limiting groove corresponding to the second liquid injection device 3 are arranged on one side of the covering member 15 close to the mounting base 11, wherein the first limiting groove includes a tubular body limiting groove 72 and an injection port limiting groove 73, and the tubular body limiting groove 72 and the injection port limiting groove 73 are adapted to the first placing groove 111. When the covering member 15 rotates to cover the mounting base 11 and the first liquid injection device 2, the second liquid injection device 3, and the triple valve 4 arranged on the mounting base 11, the tubular body limiting groove 72 cooperates with the first placing groove 111 to fix the tube body of the first liquid injection device 2, and the injection port limiting groove 73 cooperates with the first placing groove 111 to fix the injection port of the first liquid injection device 2, which avoids the movement of the tube body of the first liquid injection device 2 and ensures that the injection port of the first liquid injection device 2 and the first outlet 41 of the triple valve are connected stably. The second limiting groove has the same structure as the first limiting groove, which is not repeated herein.

As shown in FIG. 8, the covering member 15 is provided with an observation window 74. The observation window is provided in correspondence with the tube body of the liquid injection device for observing the state of the liquid in the liquid injection device, so as to facilitate the medical personnel to understand the current state of the suspension.

As shown in FIG. 5 and FIG. 7, the automatic rotating device 6 is arranged on the covering member 15. When the covering member 15 covers the mounting base 11 and the first liquid injection device 2, the second liquid injection device 3, and the triple valve 4 arranged on the mounting base 11, the automatic rotating device 6 is in contact with the valve knob 44; and when the covering member 15 exposes the mounting base 11 and the first liquid injection device 2, the second liquid injection device 3, and the triple valve 4 arranged on the mounting base 11, the automatic rotating device 6 is away from the valve knob 44.

The automatic rotating device 6 is arranged on the covering member 15, which avoids the accumulation of components on the base 1, which has a low aesthetic degree. Moreover, when the covering member 15 is in an open state (i.e., during the reagent preparation stage or at the end of the pushing stage of the foaming test), that is, when the first liquid injection device 2, the second liquid injection device 3, and the intravenous needle need a manual operation by the medical personnel, the automatic rotating device 6 is away from the valve knob 44, so as to prevent the automatic rotating device 6 from misoperation on the valve knob 44.

As shown in FIG. 5 and FIG. 7, the automatic rotating device 6 includes a power device 61 and a rotation rod 62, wherein the power device 61 is connected to the rotation rod 62, and the rotation rod 62 is used to contact the valve knob 44 and to rotate the valve knob 44 driven by the power device 61.

Specifically, the power device 61 is preferably a stepper motor. Optionally, the power device 61 can also be a servo motor.

As shown in FIG. 7, the rotation rod 62 is provided with an accommodation groove 63, wherein the accommodation groove 63 is adapted to the valve knob 44. When the covering member 15 covers the base 1, the valve knob 44 is embedded in the accommodation groove 63, and the rotation rod 62 drives the valve knob 44 to rotate synchronously under the action of the power device 61. The structure is simple and easy to operate.

As shown in FIG. 5, the piston rod of the first liquid injection device 2 is driven to move reciprocally driven by the first automatic push device 5. The first automatic push device 5 includes a first driving device 51 and a first driving rod 52, wherein the first driving device 51 is connected to the piston rod of the first liquid injection device 2 by the first driving rod 52, and the piston rod of the first liquid injection device 2 is used to drive the piston rod to move reciprocally under the drive of the first driving device 51, so that the suspension in the first liquid injection device 2 is mixed evenly.

The second automatic push device includes a second driving device and a second driving rod, wherein the second driving device is connected to the piston rod of the second liquid injection device 3 by the second driving rod, and the piston rod of the second liquid injection device 3 is used to drive the piston rod to move reciprocally under the drive of the second driving device, so that the suspension in the second liquid injection device 3 is mixed evenly.

Specifically, in the embodiment, both the first automatic push device 5 and the second automatic push device are crank-connecting rod mechanisms. Optionally, in other embodiments, a transmission structure connected by a driving device and a lead screw can also be used.

Specifically, the first driving device 51 and the second driving device are preferably stepper motors. Optionally, the first driving device 51 and the second driving device can also be servo motors.

Specifically, as shown in FIG. 8, the first driving device 51 is connected to the first driving rod 52 by the sequentially connected driving plate 53, ball-head rod end joint bearing 54, ball joint 55, and single elbow joint 56. The connection method of the second driving device and the second driving rod is the same as the connection method of the first driving device 51 and the first driving rod 52, which is not repeated herein.

Specifically, a buffer block 57 is arranged between the first driving rod 52 and the piston rod of the first liquid injection device 2 and between the second driving rod and the piston rod of the second liquid injection device 3 respectively.

As shown in FIG. 5, the base 1 is provided with a first fixing member 13 and a second fixing member 14. The first fixing member 13 has a first channel arranged in the first direction x, wherein a driving rod corresponding to the first liquid injection device 2 passes through the first channel, and the first fixing member 13 surrounds the outer side of the drive rod, so that the driving rod moves in the first direction x. The second fixing member 14 has a second channel arranged in the second direction y, wherein a driving rod corresponding to the second liquid injection device 3 passes through the second channel, and the second fixing member 14 surrounds the outer side of the drive rod, so that the driving rod moves in the second direction y.

Specifically, both the first fixing member 13 and the second fixing member 14 are composed of a bearing base and a linear ball bearing.

### Embodiment 2

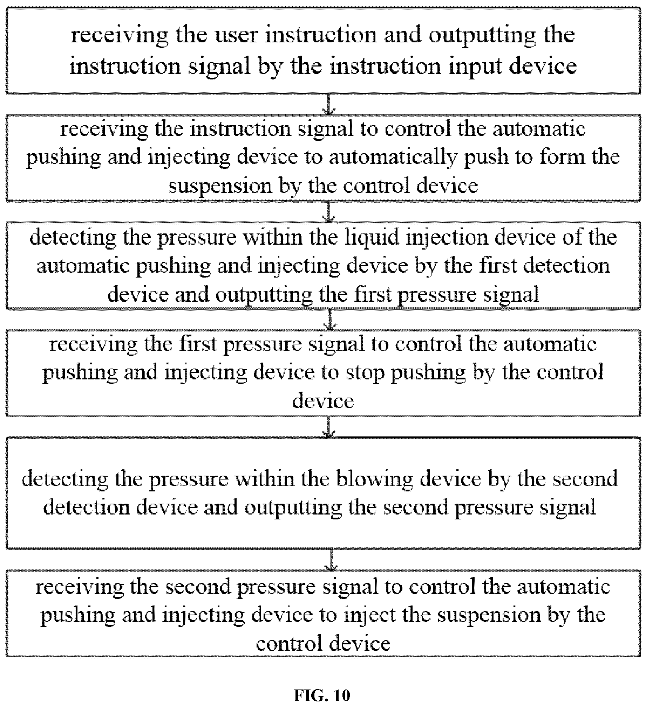

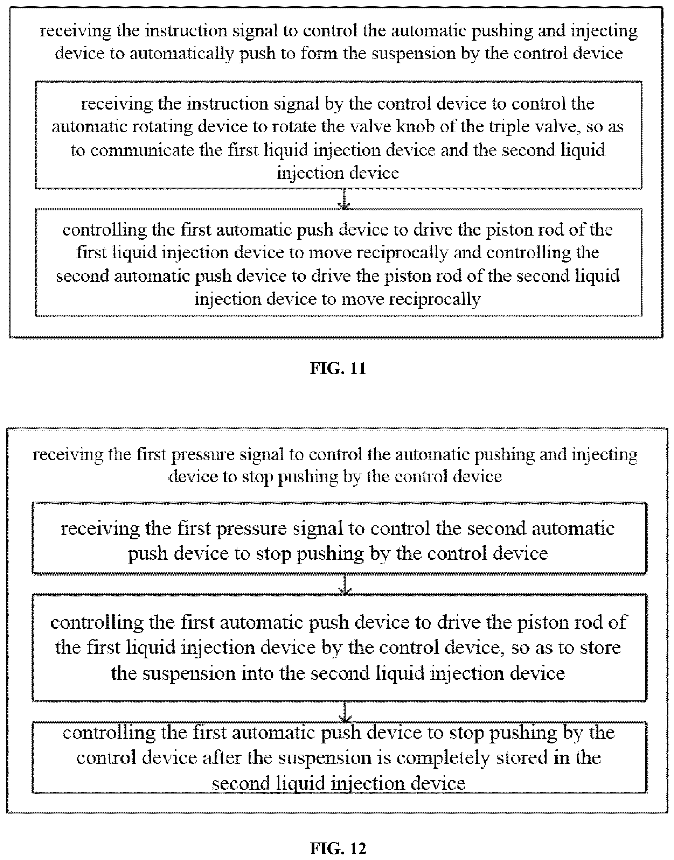

As shown in FIG. 1 to FIG. 12, a full-automatic foaming method is applied to the full-automatic foaming apparatus described in Embodiment 1, including:
receiving the user instruction and outputting the instruction signal by the instruction input device 8;
receiving the instruction signal to control the automatic pushing and injecting device to automatically push to form the suspension by the control device 9;
detecting the pressure within the liquid injection device of the automatic pushing and injecting device by the first detection device 71 and outputting the first pressure signal;
receiving the first pressure signal to control the automatic pushing and injecting device to stop pushing by the control device 9;
detecting the pressure within the blowing device by the second detection device 72 and outputting the second pressure signal; and
receiving the second pressure signal to control the automatic pushing and injecting device to inject the suspension by the control device 9.

The current foaming test needs manual reciprocal pushing of 2 syringes, so as to make a well-mixed suspension (generally pushing not less than 10 times) of the saline, air, and blood, and it needs to prepare two suspensions (one for the control group and one for the test group), so that the medical personnel needs to spend a lot of time and energy on the pushing stage, and the work intensity is high.

Moreover, the triple valve further needs to be controlled by the medical personnel during the pushing stage to communicate with the corresponding channel, so that the water, air, and blood are mixed, which requires the participation of the medical personnel during the whole process.

Furthermore, the foaming test needs the participation of at least two medical personnel (one to push and the other medical personnel to observe), which results in high labor costs.

Additionally, it is not easy to control the pushing duration through the manual operation. Due to the difference in the personal operating proficiency and operating habit of medical personnel, errors are likely to occur during the pushing process, which results in a lower diagnostic accuracy.

Moreover, some patients are unable to complete the standard Valsalva maneuver (let the patient take a deep breath, hold it, and then exhale forcefully) due to their age, which results in the incorrect diagnostic result, so that the diagnosis accuracy is not high. At present, the method of repeated diagnosis is commonly used to improve the accuracy of the diagnosis result, which is time-consuming and laborious.

The present invention provides a full-automatic foaming method, wherein the instruction input device 8, the automatic pushing and injecting device, the blowing device, the first detection device 71, the second detection device 72, and the control device 9 cooperate with each other, which realizes the full automation of the foaming process and the injection process of the foaming test and provides a high-level automation, thereby freeing hands of the medical personnel, reducing the work intensity of the medical personnel, and improving the experience of the medical personnel; and at the same time, it overcomes the defects that the diagnostic accuracy is low due to the difference in the pushing duration and the difference in the individual operation method in the manual operation, so as to improve the diagnostic accuracy rate.

As shown in FIG. 1 to FIG. 12, the step of receiving the instruction signal to control the automatic pushing and injecting device to automatically push to form the suspension by the control device 9 includes:
receiving the instruction signal by the control device 9 to control the automatic rotating device 6 to rotate the valve knob 44 of the triple valve, so as to communicate the first liquid injection device 2 and the second liquid injection device 3; and
controlling the first automatic push device 5 to drive the piston rod of the first liquid injection device 2 to move reciprocally and controlling the second automatic push device to drive the piston rod of the second liquid injection device 3 to move reciprocally by the control device 9 after the first liquid injection device 2 and the second liquid injection device 3 are communicated

As shown in FIG. 1 to FIG. 12, the step of receiving the first pressure signal to control the automatic pushing and injecting device to stop pushing by the control device 9 includes:
receiving the first pressure signal to control the second automatic push device to stop pushing by the control device 9;
controlling the first automatic push device 5 to drive the piston rod of the first liquid injection device 2 by the control device 9, so as to store the suspension into the second liquid injection device 3; and
controlling the first automatic push device 5 to stop pushing by the control device 9 after the suspension is completely stored in the second liquid injection device 3.

The pressure within the liquid injection device is detected by using the first detection device 71, which facilitates the understanding of the mixing state of the suspension, and avoids excessive pressure within the liquid injection device leading to an explosion, thereby providing a high safety coefficient.

As shown in FIG. 1 to FIG. 12, the step of receiving the second pressure signal to control the automatic pushing and injecting device to inject the suspension by the control device 9 further includes:
receiving the second pressure signal by the control device 9, wherein if the pressure value represented by the second pressure signal reaches the preset value,
controlling the automatic rotating device 6 to rotate the valve knob 44 of the triple valve by the control device 9, so as to communicate with the second liquid injection device 3 and the intravenous needle, and
controlling the second automatic push device to drive the piston rod of the second liquid injection device 3, so that the intravenous needle outputs the suspension.

It uses the blowing-sensing technology instead of requiring the user to perform the Valsalva maneuver (let the patient take a deep breath, hold it, and then exhale forcefully) to accurately assess right atrial pressure, and it is to automatically push the foaming agent when the pressure reaches the preset value, which avoids the poor test result since some patients are unable to complete the standard Valsalva maneuver due to their age, so as to provide a wide range of applications and a high accuracy of the test result. It realizes the mechanism injection without the manual participation, and the automation level is high.

It should be understood by persons of ordinary skill in the art that the modules or steps of the present invention can be realized by general purpose computing devices, and they can be concentrated on a single computing device or distributed on a network composed of multiple computing devices. Optionally, they can be realized in executable program code of the computer device, so that they can be stored in the storage device to be executed by the computing device, or they can be made into individual integrated circuit modules, or multiple modules or steps in them can be made into a single integrated circuit module. In this way, the present invention is not limited to any particular combination of hardware and software.

It is noted that the foregoing are only preferred embodiments of the present invention and the technical principles utilized. It will be understood by those skilled in the art that the present invention is not limited to the particular embodiments herein, and is capable of a variety of variations, readjustments, and substitutions apparent to those skilled in the art without departing from the scope of protection of the present invention. Therefore, although the present invention is described in more detail by the above embodiments, the present invention is not limited to the above embodiments, but may also include more equivalent embodiments without departing from the concept of the present invention, and the scope of the present invention is determined by the scope of the appended claims.

The above is only preferable embodiments of the present invention, and is not intended to limit the present invention. Any modifications, equivalent substitutions, improvements, etc. made within the spirit and principles of the present invention shall be included in the scope of protection of the present invention.

## Claims

1. A full-automatic foaming apparatus, **characterized by** comprising:
an instruction input device, configured to receive a user instruction and output an instruction signal;
an automatic pushing and injecting device, configured to automatically push to form a suspension and inject the suspension;
a blowing device, configured to receive gas exhaled by the user;
a first detection device, connected to the automatic pushing and injecting device, and configured to detect a pressure within a liquid injection device of the automatic pushing and injecting device and to output a first pressure signal;
a second detection device, connected to the blowing device, and configured to detect a pressure within the blowing device and to output a second pressure signal; and
a control device, electrically connected to the instruction input device, the automatic pushing and injecting device, the first detection device, and the second detection device; configured to receive the instruction signal and allow the automatic pushing and injecting device to push based on the instruction signal; further configured to receive the first pressure signal and allow the automatic pushing and injecting device to stop pushing based on the first pressure signal; and further configured to receive the second pressure signal and allow the automatic pushing and injecting device to inject the suspension based on the second pressure signal.

2. The full-automatic foaming apparatus according to claim 1, wherein the instruction input device comprises a start-stop button, and the start-stop button is electrically connected to the control device,
configured to receive a start instruction and to output a start instruction signal to the control device, so that the control device drives the automatic pushing and injecting device for pushing and injecting, and
further configured to receive a stop instruction and to output a stop instruction signal to the control device, so that the control device drives the automatic pushing and injecting device for stopping pushing and injecting.

3. The full-automatic foaming apparatus according to claim 1 or 2, further comprising a power source, wherein the power source is electrically connected to the control device via a switching circuit, and the instruction input device comprises a power button and an emergency stop button, wherein
the power button is electrically connected to the switching circuit, configured to receive a user power-on instruction and to output a power-on signal to conduct the switching circuit, so that the control device starts to operate, and further configured to receive a user power-off instruction and to output a power-off signal to disconnect the switching circuit, so that the control device stops operating; and
the emergency stop button is electrically connected to the switching circuit, and configured to receive a user emergency stop instruction and to output an emergency stop signal to disconnect the switching circuit, so that the control device stops operating.

4. The full-automatic foaming apparatus according to claim 1, further comprising a display device, wherein the display device is provided with an instruction signal display interface, a first pressure signal display interface, and a second pressure signal display interface; and
the display device is electrically connected to the control device, configured to receive the instruction signal output by the control device and to display the instruction signal to the instruction signal display interface, to receive the first pressure signal output by the control device and to display the first pressure signal to the first pressure signal display interface, and to receive the second pressure signal output by the control device and to display the second pressure signal to the second pressure signal display interface.

5. The full-automatic foaming apparatus according to claim 1, wherein the automatic pushing and injecting device comprises a base, and the base is provided thereon with following components:
a triple valve;
a first liquid injection device, communicated to a first outlet of the triple valve;
a second liquid injection device, communicated to a second outlet of the triple valve;
an intravenous needle, communicated to a third outlet of the triple valve;
a first automatic push device, connected to the first liquid injection device, and configured to drive a piston rod of the first liquid injection device to move reciprocally;
a second automatic push device, connected to the second liquid injection device, and configured to drive a piston rod of the second liquid injection device to move reciprocally; and
an automatic rotating device, configured to drive a valve knob of the triple valve to rotate, so as to communicate two outlets of the triple valve.

6. The full-automatic foaming apparatus according to claim 5, wherein the control device is electrically connected to the first automatic push device, the second automatic push device, and the automatic rotating device;
configured to drive the automatic rotating device to rotate, and to drive the first automatic push device and the second automatic push device to push based on the instruction signal;
further configured to drive the first automatic push device and the second automatic push device to stop pushing, and to drive the automatic rotating device to rotate based on the first pressure signal; and
further configured to drive the second automatic push device to inject the suspension based on the second pressure signal.

7. The full-automatic foaming apparatus according to claim 5, wherein the automatic rotating device comprises a power device and a rotation rod connected to each other, and the rotation rod is configured to contact the valve knob and to rotate the valve knob driven by the power device, wherein
the first automatic push device comprises a first driving device and a first driving rod, wherein the first driving device is connected to the piston rod of the first liquid injection device by the first driving rod, and
the second automatic push device comprises a second driving device and a second driving rod, wherein the second driving device is connected to the piston rod of the second liquid injection device by the second driving rod.

8. A full-automatic foaming method, applicable to the full-automatic foaming apparatus according to any one of claims 1 to 7, **characterized by** comprising:
receiving the user instruction and outputting the instruction signal by the instruction input device;
receiving the instruction signal to control the automatic pushing and injecting device to automatically push to form the suspension by the control device;
detecting the pressure within the liquid injection device of the automatic pushing and injecting device by the first detection device and outputting the first pressure signal;
receiving the first pressure signal to control the automatic pushing and injecting device to stop pushing by the control device;
detecting the pressure within the blowing device by the second detection device and outputting the second pressure signal; and
receiving the second pressure signal to control the automatic pushing and injecting device to inject the suspension by the control device.

9. The full-automatic foaming method according to claim 8, wherein the automatic pushing and injecting device comprises a base, and a triple valve, a first liquid injection device, a second liquid injection device, an intravenous needle, a first automatic push device, a second automatic push device, and an automatic rotating device arranged on the base, wherein the first automatic push device is connected to the first liquid injection device, and the first liquid injection device is communicated with a first outlet of the triple valve; the second automatic push device is connected to the second liquid injection device, and the second liquid injection device is communicated with a second outlet of the triple valve; and the intravenous needle is communicated with a third outlet of the triple valve, wherein
the step of receiving the instruction signal to control the automatic pushing and injecting device to automatically push to form the suspension by the control device comprises:
receiving the instruction signal by the control device to control the automatic rotating device to rotate a valve knob of the triple valve, so as to communicate the first liquid injection device and the second liquid injection device; controlling the first automatic push device to drive a piston rod of the first liquid injection device to move reciprocally; and controlling the second automatic push device to drive a piston rod of the second liquid injection device to move reciprocally.

10. The full-automatic foaming method according to claim 9, wherein the step of receiving the first pressure signal to control the automatic pushing and injecting device to stop pushing by the control device comprises:
receiving the first pressure signal to control the second automatic push device to stop pushing by the control device;
controlling the first automatic push device to drive the piston rod of the first liquid injection device by the control device, so as to store the suspension into the second liquid injection device; and
controlling the first automatic push device to stop pushing by the control device after the suspension is completely stored in the second liquid injection device.
